(19) **Europäisches Patentamt / European Patent Office / Office européen des brevets**

(11) **EP 1 277 433 B1**

(12) **EUROPEAN PATENT SPECIFICATION**

(45) Date of publication and mention of the grant of the patent:
**27.05.2009 Bulletin 2009/22**

(51) Int Cl.:
**A61B 5/0452** (2006.01)    **A61B 5/0205** (2006.01)

(21) Application number: **02255047.9**

(22) Date of filing: **18.07.2002**

(54) **Adjustable coefficients to customize predictive instruments**

Einstellbare Koeffizienten zur Personalisierung von prädiktiven Instrumenten

Coefficients réglables pour personnaliser des instruments prédictifs

(84) Designated Contracting States:
**AT BE BG CH CY CZ DE DK EE ES FI FR GB GR IE IT LI LU MC NL PT SE SK TR**

(30) Priority: **18.07.2001 US 306653 P**

(43) Date of publication of application:
**22.01.2003 Bulletin 2003/04**

(73) Proprietor: **NEW ENGLAND MEDICAL CENTER HOSPITALS, INC.**
**Boston, MA 02111 (US)**

(72) Inventors:
• **Selker, Harry P.**
**Wellesley,**
**Massachusetts 02481 (US)**
• **Griffith, John L.**
**Natick,**
**Massachusetts (US)**

(74) Representative: **Bassil, Nicholas Charles et al**
**Kilburn & Strode LLP**
**20 Red Lion Street**
**London WC1R 4PJ (GB)**

(56) References cited:
**US-A- 4 998 535**        **US-A- 5 724 983**

## Description

### TECHNICAL FIELD

The invention relates to predictive instruments for computing a patient's probability of a serious medical condition.

### BACKGROUND

A number of instruments have been developed that enable the physician to compute probabilities of life threatening cardiac conditions for patients. Some of these instruments are described in the following references.

A hand-held predictive instrument is described by Michael W. Pozen et al. in "A Predictive Instrument to Improve Coronary-Care- Unit Admission Practices in Acute Ischemic Heart Disease" The New England Journal of Medicine, Vol 310 pp. 1273-1278, May 17, 1984. With the handheld calculator-based instrument, a physician can compute a patient's probability of having acute cardiac ischemia based upon physician-entered values for a set of clinical variables. An automatic, computerized version of this instrument which utilizes output from a electrocardiograph and a waveform analyzer is described by H. P. Selker et al. in "A Time-Insensitive Predictive Instrument for Acute Myocardial Infarction Mortality", Med. Care 1991; 29:1196-1211.

A predictive instrument for determining the probability of acute hospital mortality of a cardiac patient is described in U.S. Pat. No. 4, 957,115 to Dr. Harry P. Selker.

The probability of acute hospital mortality is commonly understood to mean the probability of dying from a current acute condition, generally during the specific initial hospitalization for the problem. It is also know as the probability of imminent death for the patient. That is, it is a short term, as opposed to a long term, probability of mortality that does not necessarily have a precisely defined period of time associate with it.

A predictive instrument for evaluating whether to use thrombolytic therapy to treat a patient with a heart condition is described in U.S. Pat. No. 4,998,535 to Dr. Selker et al.

The predictive instrument computes a first probability of acute hospital mortality for the patient assuming that thrombolytic therapy is not administered and it computes a second probability of acute hospital mortality for the patient assuming that thrombolytic therapy is administered. The difference in the computed probabilities may assist the physician in deciding whether it would be advantageous to administer the thrombolytic therapy to the patient.

The above-mentioned predictive instruments use logistic regression equations to model the probability that the patient has a serious cardiac condition (e.g. the probability of acute cardiac ischemia or the probability of imminent death from a cardiac condition),

Such predictive instruments have also been suggested for other medical conditions or medical outcomes. See for example, U.S. Pat. No. 5,724,983 to Dr. Selker et al.

### SUMMARY

In a first aspect of the invention there is provided an apparatus for monitoring the medical condition of a patient, said apparatus comprising:

a medical instrument which during use monitors one or more clinical features of the patient; and
a predictive instrument arranged to receive output from the medical instrument and programmed to compute a probability of a medical outcome or diagnosis based on the monitored one or more clinical features, wherein the predictive instrument is further programmed to use a model to compute said probability, said model being characterized by a set of coefficients the values of which affect the computed probability; and said apparatus further characterised in that it includes
an interface through which a user enters an input characterizing at least one of a physical setting in which the predictive instrument is to be used and a sub-population of patients on which the predictive instrument is to be used, wherein said user input causes a corresponding one of a plurality of sets of coefficient values to be used as values for coefficients among the plurality of coefficients in said model.

The model may be a regression equation, an artificial feed-forward multilayer neural network, or a classification tree.

The apparatus may further comprise comprising a local data storage system storing said plurality of set of coefficient values.

The local data storage system may be RAM or disk storage.

The plurality of sets of coefficient values may include multiple sets of coefficient values, each for a different sub-population of patients.

Alternatively, the plurality of sets of coefficient values may include multiple sets of coefficient values, each for a different physical setting in which the apparatus may be used.

In a second aspect of the invention, there is provided a method for monitoring the medical condition of a patient, said method comprising:

monitoring one or more clinical features of the patient; and
computing a probability of a medical outcome or diagnosis based on the monitored one or more clinical features, said computing including:

using a model to compute the probability, said model characterized by a set of coefficients the values of which affect the computed probability; said method characterised in that said computing also includes
identifying at least one of a physical setting of the patient and a sub-population of patients of which the patient is a member,
based on the identification, selecting a corresponding one of a plurality of sets of coefficient values; and
using the selected set of coefficient values as values for coefficients among the plurality of coefficients in said model.

The model may be a regression equation, an artificial feed-forward multilayer neural network, or a classification tree.

The method may further comprise storing said plurality of sets of coefficient values in a local data storage system.

The local data storage system may be RAM or disk storage.

The plurality of sets of coefficient values may include multiple sets of coefficient values, each for a different sub-population of patients.

Alternatively, the plurality of sets of coefficient values may include multiple sets of coefficient values, each for a different physical setting in which the apparatus may be used.

Examples of how such monitoring systems could be put to advantage include use at specific hospitals where the patient population is different from the general population of all hospitals, or hospitals at which outcomes of medical care are different from what is the case at hospitals in general, or at a hospital or other care setting at which the use of medical treatments is sufficiently different than is generally the case and thus different outcomes can be expected. One could also use such a monitoring system in connection with a specific MD's medical practice which might include a patient population that is different fro a general patient population. In all of these cases, there is value in starting with a standard accepted predictive instrument and then allowing it to make its outcome predictions specifically appropriate for the patients and care of that particular setting. What is described herein is a method by which standard predictive instruments can be altered for use in special settings and in a mathematically valid and user-friendly way.

Specifically, changing by a mathematically valid method, the coefficients in a regression equation, the constant $b_0$ and/or the $b_i$ terms, or equivalent approaches in nonstandard regression methods, allows adjustment of a predictive instrument's prediction to specific uses. Typically, $b_0$ will relate to the prevalence of the disease in a particular population, and $b_i$ terms will relate to other features that relate to outcome, but there are other approaches as well. By doing this in mathematically valid ways, such as the use of the likelihood ratio to adjust for prevalence of disease to adjust $b_0$, one is able to generate valid customized versions of standard predictive instruments of medical outcomes.

Another part of this approach involves the ability of the instrument that houses the predictive model to acquire the information needed for making the required adjustments to the coefficients.

The details of one or more embodiments of the invention are set forth in the accompanying drawings and the description below. Other features, objects, and advantages of the invention will be apparent from the description and drawings, and from the claims.

## DESCRIPTION OF DRAWINGS

FIG 1 is a block diagram of a cardiac patient monitoring system that embodies the invention.
FIG 2 is a list of the coefficient values for the predictive instrument used within the system of FIG 1.
FIG 3 is a flow chart showing the operation of the system.

Like reference symbols in the various drawings indicate like elements.

**DETAILED DESCRIPTION**

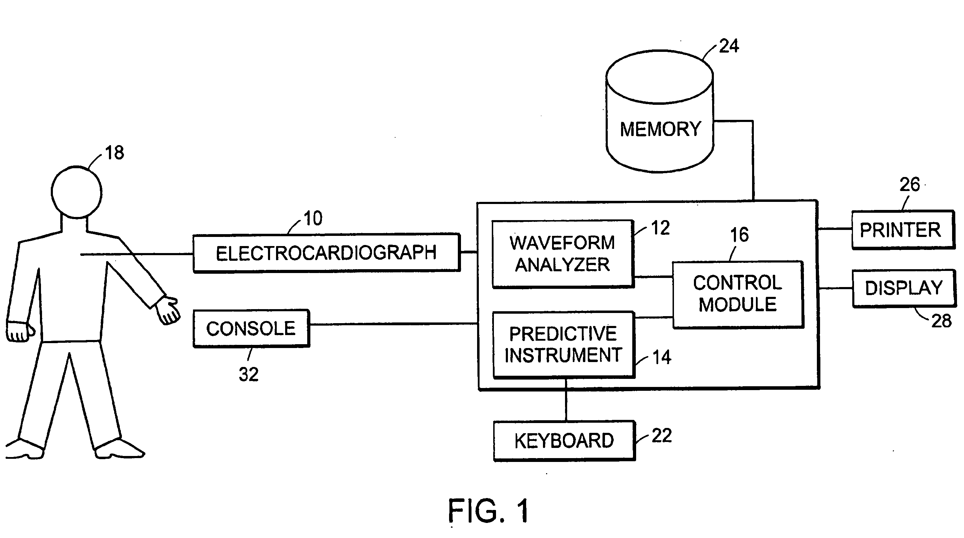

Referring to FIG. 1, a cardiac patient monitoring system, which embodies at least one aspect of the invention, includes a 12-lead electrocardiograph 10, a waveform analyzer 12, a predictive instrument 14, and a control module 16. Electrocardiograph 10 is connected to a patient 18 and produces a set of ECG waveforms for the patient. Waveform analyzer 12 is programmed to analyze the ECG waveforms and recognize the presence of certain characteristics that are particularly indicative of the cardiac condition of the patient, e.g. the presence and elevation or depression of S-T segments, the presence Q waves, and the presence of elevated, depressed or inverted T-waves. The particular characteristics that the waveform analyzer is programmed to recognize depend upon the function that is performed by the predictive instrument which in turn determines the set of clinical variables that are required to perform that function. Predictive instrument 14 uses the output of waveform analyser 12 in conjunction with other clinical information about the patient that has been entered by a physician through a keyboard 22 and computes a probability that the patient has a life-threatening cardiac condition using a stored statistical model of the condition or outcome.

Control module 16 controls the operation of the other components in the system, loads the appropriate set of coefficients into the predictive instrument from memory 24 (e.g. RAM or disk storage), and when instructed to do so updates those coefficients based on newly acquired patient data that is acquired through daily operation of the unit. Control module 16 also is responsible for printing reports out on a printer 26 attached to the system and/or displaying the report on a video screen 28.

Electrocardiograph 10 and waveform analyzer 12 are commercially available as a single unit. For example, Hewlett Packard made the HP Pagewriter XLi which is a mobile unit that can be moved from one patient to the next. The Pagewriter XLi includes a built-in 80386-based computer that can be programmed to perform the appropriate waveform analysis. That is, it can be programmed to recognize and quantify the presence of key features within the ECG waveform. It can also be programmed to identify the location of a myocardial infarction (MI) based on the characteristics of the set of signals produced by the twelve monitoring leads. Besides performing the wave analysis functions, the computer within the unit can also be programmed to perform the functions of other components or modules within the system, e.g. the computations of the predictive instrument and the functions of the control module.

In the described embodiment, predictive instrument 14 is an ACI- TIPI (Acute Cardiac Ischemia Time-Insensitive Predictive Instrument) which uses a logistic regression-based equation for computing the probability that the patient is experiencing acute cardiac ischemia. The logistic regression equation is of the form:

$$P = 100\left[1 - \frac{1}{1 + e^{b_0 + \sum b_i x_i}}\right]$$

where P is the probability of acute cardiac ischemia, $b_0$ is a constant, and the $b_i$'s are coefficients of the variables $x_i$ which are included in the model.

The system comes with a standard model of the general patient population in general. The model is in the form of a logistic regression equation with corresponding values for the coefficients.

Standard, well known regression techniques may be employed to identify the most appropriate set of explanatory variables, namely, the x; 's, and to determine the values of the coefficients of these variables. For a description of such techniques and examples of commercially available computer programs that implement them, see N. C. Cary in SUGI Supplement Library User's Guide, SAS Institute, p. 181 - 202, 1983, and L. Engelman, "PLR Stepwise Logistic Regression," BMDP Statistical Software, Chap. 14.5, pp. 330-334, BMDP publishers, Westwood, Calif. Of course, the precise set of explanatory variables that are identified and the predictive ability of the resulting logistic equation generally depends upon the quality of the underlying data that is used to develop the model. Such factors as the size and completeness of the database are often of significant importance. The selection of the relevant variables and the computation of the appropriate coefficients are well within the skill of an ordinary person skilled in the art.

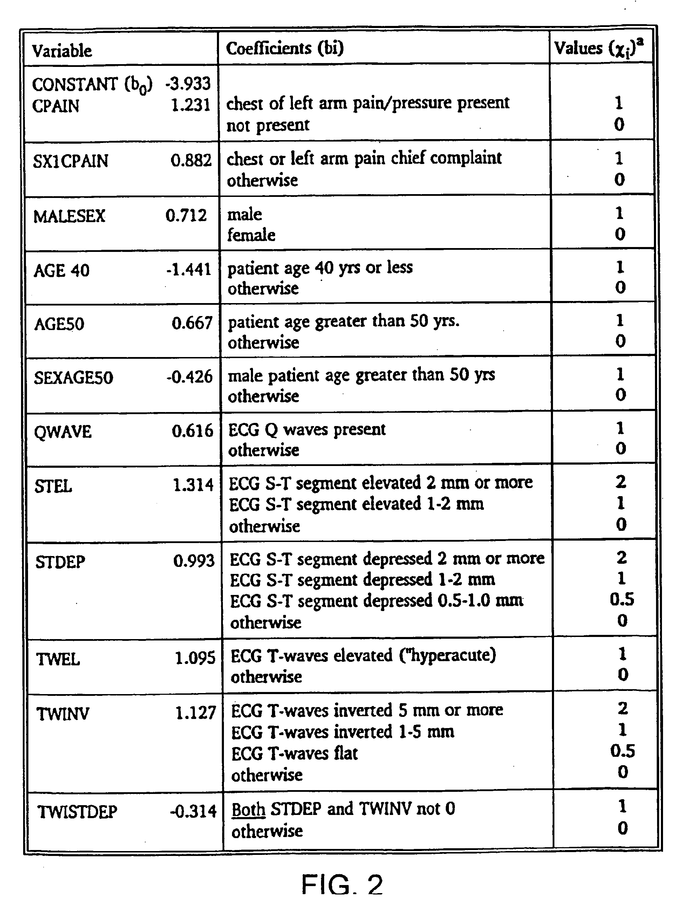

The variables that are used in this equation are shown in FIG. 2 along with the values of the coefficients and the values which the x; 's can take for the different variables. Note that only the largest value for x is used per variable. Also ECG findings must be present in at least two leads, and S-T segment and T wave changes are "normal" if secondary to right or left complete bundle branch blocks, left ventricular hypertrophy, or a paced QRS. Only one type of abnormality is coded each for S-T segment and for T wave per patient (exclusive of TWISTDEP), use with elevation taking priority. Deviations are expressed in mm using the standard ECG scale of 1 mm=0.1 mV.

The system may also include multiple sets of coefficients for sub-populations of patients defined either in terms of the physical setting in which the system is being used (e.g. ambulance, emergency room, patient ward, etc.) and/or in terms of particular characteristics of the sub-population of patients (e.g. elderly, women, African American, white male, etc.)

These other sets of coefficients are derived from set of coefficients for the general population by using further new data acquired from that sub-population. In other words, the same regression equation is used for the sub-population and only one or more of the base set of coefficients is modified on the basis of the new patient data.

To generate the coefficients for the tailored models, one need not derive and construct a new model from scratch, as was done when the original model was produced. The process to modify the existing predictive equation to produce a tailored equation (i.e., a model with revised coefficients) is computationally much simpler than the process that was required to generate the original general equation. That is, it avoids the very computationally and data intensive project that was required to generate the original equation. But still one obtains the robustness of the original model but customized for the population to which it will be applied.

There are known techniques and shortcuts for modifying previously computed coefficients in view of new data. Recalculating coefficients is relatively simple, especially in comparison to the effort and data that is required to develop the original model. This is because the model and the explanatory variables are already known - having been identified from the earlier analysis. The techniques for revising coefficients in view of newly acquired date are well known to person of skill in the art. Articles describing these techniques can be readily found in the publicly available literature. See for example, the following references: Medical Decision Making, "Neural Networks in Clinical Medicine," Will Penny et al. Vol. 16, No. 4 (Oct.-Dec. 996), pp. 386-398; Parallel Distributed Processing: Explorations in the Microstructure of Cognitions, Volume 1: Foundations, "An Analysis of the Delta Rule and the Learning of Statistical Associations," G.O. Stone, Chap. 11, pp. 444-459 (1988); Parallel Distributed Processing: Explorations in the Microstructure of Cognition, Volume 1:, Foundations, "Learning Internal Representations by Error Propagation," D.E. Rumelhart et al., Chap, 8, pp. 318 et seq. (1988); and Regression and the Moore-Penrose Pseudoinverse, "Recursive Computation of Least Squares Estimators," Chap. VIII, Academic Press (1972).

To make these calculations, one needs to also store in memory the historical data representative of the computations that were done to arrive at the initial set of coefficients. This history is in the form of correlations between the various explanatory variables. It is basically a summary of the data that were used to derive the original model and its coefficients.

One selects the coefficients for the explanatory variables that are expected to be influenced the most by limiting the patients to a sub-population. And for that selected group, the values of the coefficients is computed. Of course, the simplest approach is to only modify the intercept or the value of the constant coefficient. But, as noted, in addition to modifying the intercept, one could also modify coefficients for other variables that reflect what one knows about the population. For example, if the population is primarily of elderly patients, the coefficients of the age variable and/or the gender variable might be appropriate ones to modify.

Typically, one weights the new observations, i.e., giving them additional and potentially more weight than the original observations from which the model was derived. The weights determine by how much the computed values based on the revised coefficients change.

The sets of coefficients can be generated in one of several different locations. In the described embodiment, the computing of new coefficients is done locally to the instrument. That is, as the monitoring system is being used on each patient, the system stores in memory the data that are inputted for each patient including the clinical data inputted through the keyboard as well as the monitoring data from the monitoring instrument The predictive instrument computes a probability and the treating physician or operator takes whatever action is appropriate in view of the computed probability. At some later time, the actual outcome as compared to the probabalistic outcome, is known, and that information is also entered into the system by somebody as part of a stored record for the patient. When a sufficient number of new patient records have been stored in memory, the operator or administrator causes the instrument to compute a revised set of coefficients for the sub-population of which the new patient records are representative. The computing of revised coefficients can be performed with as few as one new patient record or as many as are available. Of course, the more data used in computing the revised coefficients, the more robust will be the results.

Alternatively, the process of computing the revised coefficients can be performed at a central location that is remote from the individual monitoring systems and the results can then be downloaded into all of the appropriate systems for use locally. This would have the advantage of taking advantage of a larger set of data and in producing multiple monitoring systems that are programmed identically.

In any case, regardless of how or where the revised coefficients are computed, the result is a monitoring system which stores multiple sets of coefficients, each set for a different sub-population of patients on which the system is likely to be used.

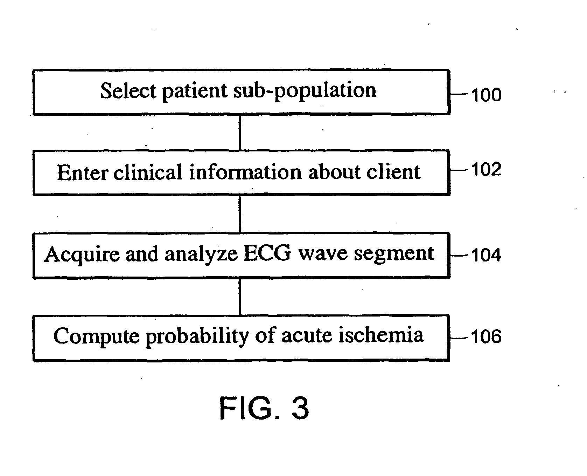

The system is programmed to operate in the manner shown in FIG. 3. When the system is first used, the operator selects, from among a number of choices presented to him, the particular setting in which it is being used and/or the particular sub-population of patients on which it is being used (step 100). In response to this selection, the controller loads a corresponding set of coefficients into the model thereby tailoring the mode] to that particular setting and/or sub-population. When the patient is first connected to the system, the physician enters the relevant clinical information about the patient (step 102). For example, if the predictive instrument is programmed to compute the probability of acute cardiac ischemia in accordance with the above model, the physician enters the following information about the patient: (1) name; (2) age; (3) sex; (4) whether the patient is experiencing chest or left arm pain; and (5) whether the patient's chief complaint is chest or left arm pain. After the physician has set up the system for a particular patient and connects the leads of the electrocardiograph to the patient, the physician causes the system to perform an initial ECG for the patient. In response, the waveform analyzer acquires and analyzes a current segment of the patient's ECG waveform (step 104). Typically, a 10-20 second segment of the patient's ECG is required by the waveform analyzer to perform its waveform analysis functions. The output of the waveform analyzer passes to the predictive instrument, which may, for example, be implemented by a computation routine within the computer. In the present embodiment, the output of the waveform analyser reports whether: (1) any Q waves are present; (2) whether the S- T segment is elevated or depressed and by how much; (3) whether the T- waves are elevated inverted or flat; (4) if the T-waves are elevated, by how much; and (5) whether both the STDEP and TWINV leads are non- zero.

Using the output of the waveform analyzer and previously entered values for other clinical variables, the predictive instrument computes a probability that the patient has acute cardiac ischemia (step 106).

The logistic regression equation presented above is presented as merely illustrative of one way in which the cardiac condition and the probability can be modeled. There are a variety of statistical methods and algorithms that can be used for calculating the predicted probability of an adverse cardiac event or a life threatening cardiac condition. These include, for example, artificial feed-forward multilayer neural networks and classification trees. Although the procedures may differ among these various techniques, many of them may generally be expressed in terms of finding an optimal solution to the following general equation:

$$f^{-1}(probability\ event) = \beta_0 + \sum_{l=1}^{K} \beta_l g_l(X_1, X_2, ... X_p)$$

where $f^{-1}$ and $g_i$ are general functions, the $X_n$'s (where $1 \leq n \leq p$) are the values of the independent input variables, and $\beta_0$ and $\beta_i$ (where $I \leq i \leq K$) are model coefficients,

The standard logistic regression equation described earlier can be derived from the above equation. In addition an artificial network with a single hidden layer and an activation function "h" can be written as:

$$f^{-1}(output) = \beta_0 + \sum_{i=1}^{K} \left\{ \beta_i h\left( \sum_{j=1}^{p} \alpha_{ij} X_j \right) \right\}$$

Other embodiments are within the following claims. Even though computing the probability of acute cardiac ischemia was used as an example, this was not meant to limit the scope of the invention to only those systems which compute that particular probability. The invention also encompasses systems which compute any probability of a serious cardiac condition. In addition, the method by which the probability is computed is not central to the invention. Any approach which attempts to model historical patient information to compute a probability of the patient having a serious cardiac condition based at least in part upon ECG information falls within the scope of this invention.

In addition, a predictive instrument has applicability to many other medical problems beyond the above-described cardiac related problems. The extension to circumstances that are not cardiac related is straightforward. For example, in the domain of pulmonary medicine, a patient in an intensive care unit could be under observation for potential respiratory failure. The classical approach is to use on-going single function monitors of indicators such as the patient's heart rate, respiratory rate, and perhaps under some circumstances, blood oxygen saturation. As with the cardiac situation, however, any single one of these parameters has limited utility because of its possible contamination by noise and because of its inherent inability to be a good proxy for the wide variety of clinical features and other factors that are really needed to provide an accurate estimate of the probability of the medical outcome in question. On the other hand, the mathematical model described herein (e.g., logistic regression) that takes into account the heart rate, blood pressure, respiratory rate, blood oxygen saturation level, and perhaps other clinical features, will give a far more accurate indication of and prediction of true respiratory failure. The other clinical features include for example the patient's age, symptoms (e.g. shortness of breath), and the patient's past medical history (e.g., history of smoking, history of prior respiratory failure, etc.). In other words, the mathematical model wil give a more accurate and usable (e.g. noise-free) estimate of the patient's condition.

A further example in the pulmonary area may be useful. In the case of imminent respiratory failure, a small increase in respiratory rate and heart rate would likely be missed, or might be overwhelmed by noise, or even if detected might be viewed as insignificant. However, if measured in conjunction with other clinical features, including for example a small decrease in oxygen saturation, these observations when included in a logistic regression (or other mathematical model) will be multiplicative, and thus, give a substantial (accurate and specific) increase in the computed probability of respiratory failure. Thus, since the mathematical model takes multiple factors into account, as in the cardiac monitor situation, it will provide an opportunity to early on detect a change in the computed probability value with sufficient fidelity in the real-time setting to support targeted clinical intervention.

In a generalized monitoring system, the electrocardiograph 10 is any device for real-time monitoring of one or more clinical features of the and predictive instrument is a generalized predictive instrument for computing a probability of a medical outcome or diagnosis, based in part on those monitored clinical features. The monitoring device could be a single instrument, as it was in the case of the above-described cardiac monitor which used an electrocardiograph, or it could be a constellation of medical instruments, as will bc the case in some of the examples systems described below.

As previously described in connection with the cardiac monitoring system, the generalized predictive instrument uses the output of monitoring device in conjunction with other clinical information about the patient that has been entered by a physician through an input device (e.g. keyboard, network connection, database program, etc.) and computes a probability of a particular medical outcome or diagnosis for the patient.

In the described embodiments, the probability of the medical diagnosis or medical outcome is computed using a logistic regression equation of the following general form:

$$P = 100\left[1 - \frac{1}{1 + e^z}\right]$$

where P is the probability of a particular medical outcome or diagnosis, and where z has the following general form:

$$z = b_0 + \sum b_i x_i$$

In this equation, $b_0$ is a constant, and the $b_i$'s are coefficients of the explanatory variables $x_i$ which are included in the model. The variables that are used in the model will of course depend upon the particular medical outcome or diagnosis which is being evaluated. Some examples are presented below along with variables that would be included in the model.

As noted above in connection with the cardiac instrument, mathematical models other than the logistic regression equation can of course be employed. The invention is not limited to the use of a logistic regression equation to model the probability of a particular medical outcome or diagnosis.

The following are further examples of predictive instruments for non-cardiac diagnoses or medical outcomes, along with their likely key clinical variables.

In the domain of vascular disorders, a predictive instrument for pulmonary embolus includes as key variables the same variables as listed above, as well as a modification of the symptoms regarding the suddenness of onset, and also ECG variables related to right heart strain due to obstruction of the pulmonary artery (e.g. relating to QRS complex and ST/T wave changes).

In the domain of neurologic disorders, a predictive instrument for cerebral hemorrhage (i.e., hemorrhagic stroke) includes as key variables the patient's history of medications (e.g. anticoagulants, etc.), prior history of cardiac disorders that predispose to cerebral thrombosis (e.g. atrial fibrillation, valvular disease), and history of trauma to the head, as well as laboratory tests such as prothrombin time and partial thromboplastin time and platelet count, which are all measures of the clotting ability of the blood. They also include blood pressure, with emphasis on pulse pressure (i.e., a difference between the systolic and diastolic pressure). Data from an imaging machine (e.g. CT or MRI scan) might also be used.

In the domain of general surgery, a predictive instrument for intra-abdominal catastrophe (i.e., the need for emergency surgery) includes as key variables the presence of abdominal pain, the patient's physical findings by the physician of so-called peritoneal signs (rigidity of the abdomen, no bowel sounds, etc.), monitor data such as temperature, heart rate, blood pressure, and potentially x-ray findings that reveal bowel gas pattern.

In the domain of infectious diseases, a predictive instrument for sepsis (i.e., overwhelming infection that would require certain emergency treatments) includes as key variables the patient's level of consciousness, specific complaints, and monitored physiologic measures such as blood pressure (especially low), heart rate (especially high), respiratory rate (especially high), body temperature (especially cither unduly high or unduly low), ECG abnormalities (as not showing a contrary cardiac cause for derangements in heart rate, blood pressure, respiratory rate and other findings in the form of an interaction term in the mathematical model) laboratory tests such as white blood count (either unduly high or unduly low), interleukin levels, and other special blood tests typically available on-line from hospital clinical information systems.

Also, other ongoing monitoring data relating to blood and other body fluid culture results, such as are typically available on electronic clinical information systems, are further important explanatory variables relating to the source of sepsis.

Each of the above diagnoses to be predicted have consequences in terms of clinical outcomes including, for example, mortality. Other outcomes that can be predicted by a predictive instrument include mortality due to acute myocardial infarction or congestive heart failure, as well as conditional outcomes such as are obtained from the thrombolytic predictive instrument (see U.S. Pat. No. 4,998,535). The use of this approach also applies to other non-cardiac conditions for which prediction of medical outcomes can be conditioned on the use of specific therapies.

The examples presented above are meant to be merely illustrative of the many ways in which a predictive instrument can be used to evaluate a patient's medical condition. It is not intended that the invention be limited to the few examples that were described here. In general, the predictive instrument is any instrument which computes, on the basis of a set of clinical features, a probability of a medical outcome or diagnosis. It is intended that the invention cover the use of such predictive instruments to evaluate any medical condition that lends itself to such monitoring, regardless of the particular set of clinical features and regardless of the particular medical outcome or diagnosis.

By clinical features, it should be understood that we mean any data form that gives direct information about the clinical, i.e., the medical, state of patient. Some of the variables used in the above descriptions have focused on circumstances in which clinical features can be directly acquired in electronic form, such as heart rate and respiratory rate acquired by monitors, such as waveforms acquired by an electrocardiograph, such as oxygen saturation measured by a sensor attached to the patient's finger, and such as biochemical laboratory results. However, other clinical features can be obtained in other forms and in other ways as well. For example, as described above in connection with the use of the cardiac predictive instrument, direct questioning of the patient provides important clinical features, namely, whether or not the patient is having chest pain and whether chest pains are the chief complaint. Analogously, for a predictive instrument for predicting various respiratory diagnoses or outcomes, a relevant clinical feature would be an indication as to whether the patient has shortness of breath. These symptoms are linked to the relevant underlying medical processes and can, in combination with other clinical variables in the mathematical model, add important information. And in the case of a predictive instrument for predicting neurologic problems, relevant clinical features include indications as to whether the patient has a headache and/or neurologic symptoms. In addition, some sources of data that are less directly connected to the patient but nevertheless still represent important clinical features. For example, sociodemographic data such as age and gender are obviously important, as was the case for the cardiac predictive instrument described above. In addition, other relevant clinical feature data is reflected in medical insurance claims, such as the mere performance of certain tests, the fact of hospitalization, the actual diagnostic code (e.g. the ICD9 Code), etc. A clinical feature is any piece of additional information about the patient, which when incorporated into a mathematical model predicts a given medical outcome.

A medical outcome or medical diagnosis, such as might be predicted by a predictive instrument, is defined in the following way. A medical outcome is the state of a patient defined in medical terms, typically described in the context of a particular constellation of presenting symptoms and clinical features. In practice, the outcome is selected to be clinically meaningful to the care of the patient. Therefore, an important medical outcome for a patient with a heart attack is mortality. For a person with respiratory failure, it is also mortality but it is also long-term respiratory disability, which might be defined, for example, as lack of ability to do activities of daily living due to shortness of breath as well. For a person with neurologic presenting symptoms, an important medical outcome is preservation of normal mental function as well as mortality.

There is overlap between the concept of medical outcomes and medical diagnoses. For example, when a decision has to be made about a patient, before the clinician can evaluate the likely ultimate outcome, the clinician must first consider the specific medical diagnosis or family of diagnoses that the patient has which require attention. Thus, a diagnosis is, in a sense, an intermediate "outcome". For a person coming to the emergency department with chest pain and/or other signs and symptoms, the first question on the clinician's mind is, what is the diagnosis? If the diagnosis, i.e., the ongoing medical/clinical process, is acute cardiac ischemia (i.e., occlusion of a coronary artery), then this requires treatment to prevent its potential downstream outcomes from occurring (e.g., death) and that treatment is very different from what would be used if the diagnosis is costochondritis (inflammation of the joints between the ribs and sternum), stomach ulcer, or some other cause of the symptoms. Moreover, for all the medical conditions alluded to above, detecting through continuous monitoring of the patient changes in the likelihood of such diagnoses can have very important clinical implications for treatments. That is, very different treatments are needed for acute cardiac ischemia as compared to an ulcer, for respiratory failure as compared to a cold, or for a rupture of a cerebral aneurism as compared to a tension headache. Thus, it can be critically important to detect when the probability of a medical outcome changes.

From the above, it should be apparent that the invention can be used with a wide variety of patient monitoring devices including, for example, heart rate monitors, respiratory rate monitors, bioanalyzers, blood oximeters, and fetal monitors, just to name a few.

A number of embodiments of the invention have been described.

For example, the system embodying the invention can update the coefficients one step at a time as each new patient is monitored and the actual outcome is known. In this alternative system, the machine periodically updates the coefficients in view of new patient data so that over time the values of the coefficients slowly evolve to reflect what is occurring in the particular environment in which the machine is being used. In addition, rather than simply changing the coefficients for the model that is stored in the monitoring system, different models can be stored, each one for a different sub-population, as defined above.

Furthermore, rather than storing the model and the coefficients in the monitoring system, either the model, or the coefficients or both can be stored remotely and accessed when needed.

Accordingly, other embodiments are within the scope of the following claims.

**Claims**

1. An apparatus for monitoring the medical condition of a patient, said apparatus comprising:

   a medical instrument which during use monitors one or more clinical features of the patient; and
   a predictive instrument arranged to receive output from the medical instrument and programmed to compute a probability of a medical outcome or diagnosis based on the monitored one or more clinical features, wherein the predictive instrument is further programmed to use a model to compute said probability, said model being **characterized by** a set of coefficients the values of which affect the computed probability; and said apparatus further **characterised in that** it includes
   an interface through which a user enters an input characterizing at least one of a physical setting in which the predictive instrument is to be used and a sub-population of patients on which the predictive instrument is to be used, wherein said user input causes a corresponding one of a plurality of sets of coefficient values to be used as values for coefficients among the plurality of coefficients in said model.

2. The apparatus of claim 1, wherein the model is selected from the group consisting of a regression equation, an artificial feed-forward multilayer neural network, and a classification tree.

3. The apparatus of claim 1 or 2, further comprising a local data storage system storing said plurality of set of coefficient values.

4. The apparatus of claim 3, wherein the local data storage system is RAM or disk storage.

5. The apparatus of any preceding claim, wherein the plurality of sets of coefficient values includes multiple sets of coefficient values, each for a different sub-population of patients.

6. The apparatus of any preceding claim, wherein the plurality of sets of coefficient values includes multiple sets of coefficient values, each for a different physical setting in which the apparatus may be used.

7. A method for monitoring the medical condition of a patient, said method comprising:

   monitoring one or more clinical features of the patient; and
   computing a probability of a medical outcome or diagnosis based on the monitored one or more clinical features, said computing including:

   using a model to compute the probability, said model **characterized by** a set of coefficients the values of which affect the computed probability; said method **characterised in that** said computing also includes identifying at least one of a physical setting of the patient and a sub-population of patients of which the patient is a member,
   based on the identification, selecting a corresponding one of a plurality of sets of coefficient values; and using the selected set of coefficient values as values for coefficients among the plurality of coefficients in said model.

8. The method of claim 7, wherein the model is selected from the group consisting of a regression equation, an artificial feed-forward multilayer neural network, and a classification tree.

9. The method of claim 7 or 8, further comprising storing said plurality of sets of coefficient values in a local data storage system.

10. The method of claim 9, wherein the local data storage system is RAM or disk storage.

11. The method of any one of claims 7 to 10, wherein the plurality of sets of coefficient values includes multiple sets of coefficient values, each for a different sub-population of patients.

12. The method of any one of claims 7 to 11, wherein the plurality of sets of coefficient values includes multiple sets of coefficient values, each for a different physical setting in which the apparatus may be used.

**Patentansprüche**

1. Vorrichtung zum Überwachen des medizinischen Zustands eines Patienten, wobei die Vorrichtung umfasst:

   ein medizinisches Instrument, welches in Verwendung ein oder mehrere klinische Merkmale des Patienten überwacht; und
   ein prädiktives Instrument, das angeordnet ist, um eine Ausgabe von dem medizinischen Instrument zu empfangen und das programmiert ist, um eine Wahrscheinlichkeit einer medizinischen Folge oder Diagnose basierend auf dem überwachten Merkmal oder auf den überwachten Merkmalen zu berechnen, wobei das prädiktive Instrument weiters programmiert ist, um zur Berechnung der Wahrscheinlichkeit ein Modell zu verwenden, wobei dieses Modell durch einen Satz von Koeffizienten **gekennzeichnet** ist, deren Werte die berechnete Wahrscheinlichkeit beeinflussen; und wobei die Vorrichtung weiters **dadurch gekennzeichnet ist, dass** sie eine Schnittstelle enthält, über welche ein Benutzer eine Eingabe tätigt, die zumindest eines von physikalischem Setting, in dem das prädiktive Instrument zu verwenden ist, und einer Unterpopulation von Patienten, auf der das prädiktive Instrument zu verwenden ist, kennzeichnet, wobei die Benutzereingabe einen entsprechenden Satz einer Mehrzahl von Sätzen von Koeffizientenwerten bewirkt, die als Werte für die Koeffizienten innerhalb der Mehrzahl von Koeffizienten im Modell zu benutzen sind.

2. Vorrichtung nach Anspruch 1, wobei das Modell aus der Gruppe bestehend aus Regressionsgleichung, künstlichem vorwärtsgekoppelten, mehrschichtigen neuronalen Netz und einem Klassifizierungsbaum ausgewählt ist.

3. Vorrichtung nach Anspruch 1 oder 2, weiters mit einem lokalen Datenspeichersystem, das die Mehrzahl von Sätzen von Koeffizientenwerten speichert.

4. Vorrichtung nach Anspruch 3, wobei das lokale Datenspeichersystem ein RAM oder ein Plattenspeicher ist.

5. Vorrichtung nach einem vorhergehenden Anspruch, wobei die Mehrzahl von Sätzen von Koeffizientenwerten mehrfache Sätze von Koeffizientenwerten enthält, jeden für eine unterschiedliche Unterpopulation von Patienten.

6. Vorrichtung nach einem vorhergehenden Anspruch, wobei die Mehrzahl von Sätzen von Koeffizientenwerten mehrfache Sätze von Koeffizientenwerten enthält, jeden für ein unterschiedliches physikalisches Setting, in dem die Vorrichtung verwendet werden kann.

7. Verfahren zum Überwachen des medizinischen Zustands eines Patienten, wobei das Verfahren umfasst:

   das Überwachen eines oder mehrerer klinischer Merkmale des Patienten; und
   das Berechnen einer Wahrscheinlichkeit einer medizinischen Folge oder Diagnose basierend auf dem einen überwachten Merkmal oder auf den mehreren überwachten Merkmalen, wobei das Berechnen miteinschließt:
   das Verwenden eines Modells zur Berechnung der Wahrscheinlichkeit, wobei das Modell durch einen Satz von Koeffizienten **gekennzeichnet** ist, deren Werte die berechnete Wahrscheinlichkeit beeinflussen; wobei das Verwenden **dadurch gekennzeichnet ist, dass** das Berechnen ebenfalls miteinschließt:
   das Identifizieren zumindest eines von physikalischem Setting des Patienten und einer Unterpopulation von Patienten, zu denen der Patient gehört;
   das Auswählen eines entsprechenden Satzes einer Mehrzahl von Sätzen von Koeffizientenwerten basierend auf der Identifizierung; und
   das Verwenden des ausgewählten Satzes von Koeffizientenwerten als Werte für Koeffizienten innerhalb der Mehrzahl von Koeffizienten im Modell.

8. Verfahren nach Anspruch 7, wobei das Modell aus der Gruppe bestehend aus Regressionsgleichung, künstlichem vorwärtsgekoppelten, mehrschichtigen neuronalen Netz und einem Klassifizierungsbaum ausgewählt ist.

9. Verfahren nach Anspruch 7 oder 8, weiters umfassend das Speichern der Mehrzahl von Sätzen von Koeffizientenwerten in einem lokalen Datenspeichersystem.

10. Verfahren nach Anspruch 9, wobei das lokale Datenspeichersystem ein RAM oder ein Plattenspeicher ist.

11. Verfahren nach einem der Ansprüche 7 bis 10, wobei die Mehrzahl von Sätzen von Koeffizientenwerten mehrfache Sätze von Koeffizientenwerten enthält, jeden für eine unterschiedliche Unterpopulation von Patienten.

12. Verfahren nach einem der Ansprüche 7 bis 11, wobei die Mehrzahl von Sätzen von Koeffizientenwerten mehrfache Sätze von Koeffizienten enthält, jeden für ein unterschiedliches physikalisches Setting, in dem die Vorrichtung verwendet werden kann.

## Revendications

1. Appareil de surveillance de l'état médical d'un patient, ledit appareil comprenant :

un instrument médical qui, au cours de son utilisation, surveille une ou plusieurs caractéristiques cliniques du patient ; et
un instrument prédictif agencé pour recevoir la sortie provenant de l'instrument médical et programmé pour calculer une probabilité d'un résultat ou diagnostique médical en se basant sur une ou plusieurs caractéristiques cliniques surveillées, dans lequel l'instrument prédictif est programmé en outre pour utiliser un modèle pour calculer ladite probabilité, ledit modèle étant **caractérisé par** un groupe de coefficients dont les valeurs affectent la probabilité calculée ; et ledit appareil étant **caractérisé en ce qu'**il comporte
une interface par l'intermédiaire de laquelle un utilisateur saisit une entrée caractérisant au moins un réglage physique dans lequel l'instrument prédictif doit être utilisé et une sous-population de patients sur laquelle l'instrument prédictif doit être utilisé, dans lequel ladite saisie de l'utilisateur amène un groupe de valeurs de coefficient correspondantes parmi une pluralité de groupes de valeurs de coefficient à être utilisées comme valeurs pour des coefficients parmi la pluralité de coefficients dans ledit modèle.

2. Appareil selon la revendication 1, dans lequel le modèle est sélectionné parmi le groupe constitué d'une équation de régression, d'un réseau neuronal bouclé multicouches artificiel, et d'un arbre de classification.

3. Appareil selon la revendication 1 ou 2, comprenant en outre un système de stockage de données locales stockant ladite pluralité de groupes de valeurs de coefficient.

4. Appareil selon la revendication 3, dans lequel le système de stockage de données locales est une mémoire RAM ou une mémoire à disque.

5. Appareil selon l'une quelconque des revendications précédentes, dans lequel la pluralité de groupes de valeurs de coefficient comporte de multiples groupes de valeurs de coefficients, chacun pour une sous-population différente de patients.

6. Appareil selon l'une quelconque des revendications précédentes, dans lequel la pluralité de groupes de valeurs de coefficient comporte de multiples groupes de valeurs de coefficient, chacun pour un réglage physique différent dans lequel l'appareil peut être utilisé.

7. Procédé de surveillance de l'état médical d'un patient, ledit procédé comprenant les étapes consistant à :

surveiller une ou plusieurs caractéristiques cliniques du patient ; et
calculer une probabilité d'un résultat ou diagnostique médical en se basant sur la ou les caractéristiques cliniques surveillées, ledit calcul comportant l'étape consistant à :

utiliser un modèle pour calculer la probabilité, ledit modèle étant **caractérisé par** un groupe de coefficients dont les valeurs affectent la probabilité calculée ; ledit procédé étant **caractérisé en ce que** ledit calcul comporte également les étapes consistant à

identifier au moins un parmi un réglage physique du patient et une sous-population de patients dont le patient est un élément,

en se basant sur l'identification, sélectionner une valeur correspondante parmi une pluralité de groupes de valeurs de coefficient ; et

utiliser le groupe sélectionné de valeurs de coefficient comme valeurs pour des coefficients parmi la pluralité de coefficients dans ledit modèle.

8. Procédé selon la revendication 7, dans lequel le modèle est sélectionné parmi le groupe constitué d'une équation de régression, d'un réseau neuronal multicouches bouclé artificiel, et d'un arbre de classification.

9. Procédé selon la revendication 7 ou 8, comprenant en outre l'étape consistant à stocker ladite pluralité de groupes de valeurs de coefficient dans un système de stockage de données locales.

10. Procédé selon la revendication 9, dans lequel le système de stockage de données locales est une mémoire RAM ou une mémoire à disque.

11. Procédé selon l'une quelconque des revendications 7 à 10, dans lequel la pluralité de groupes de valeurs de coefficient comporte de multiples groupes de valeurs de coefficient, chacun pour une sous-population différente de patients.

12. Procédé selon l'une quelconque des revendications 7 à 11, dans lequel la pluralité de groupes de valeurs de coefficient comporte de multiples groupes de valeurs de coefficient, chacun pour un réglage physique différent dans lequel l'appareil peut être utilisé.

FIG. 1

EP 1 277 433 B1

| Variable | | Coefficients (bi) | Values $(\chi_i)^a$ |
|---|---|---|---|
| CONSTANT ($b_0$) | -3.933 | | |
| CPAIN | 1.231 | chest of left arm pain/pressure present | 1 |
| | | not present | 0 |
| SX1CPAIN | 0.882 | chest or left arm pain chief complaint | 1 |
| | | otherwise | 0 |
| MALESEX | 0.712 | male | 1 |
| | | female | 0 |
| AGE 40 | -1.441 | patient age 40 yrs or less | 1 |
| | | otherwise | 0 |
| AGE50 | 0.667 | patient age greater than 50 yrs. | 1 |
| | | otherwise | 0 |
| SEXAGE50 | -0.426 | male patient age greater than 50 yrs | 1 |
| | | otherwise | 0 |
| QWAVE | 0.616 | ECG Q waves present | 1 |
| | | otherwise | 0 |
| STEL | 1.314 | ECG S-T segment elevated 2 mm or more | 2 |
| | | ECG S-T segment elevated 1-2 mm | 1 |
| | | otherwise | 0 |
| STDEP | 0.993 | ECG S-T segment depressed 2 mm or more | 2 |
| | | ECG S-T segment depressed 1-2 mm | 1 |
| | | ECG S-T segment depressed 0.5-1.0 mm | 0.5 |
| | | otherwise | 0 |
| TWEL | 1.095 | ECG T-waves elevated ("hyperacute) | 1 |
| | | otherwise | 0 |
| TWINV | 1.127 | ECG T-waves inverted 5 mm or more | 2 |
| | | ECG T-waves inverted 1-5 mm | 1 |
| | | ECG T-waves flat | 0.5 |
| | | otherwise | 0 |
| TWISTDEP | -0.314 | Both STDEP and TWINV not 0 | 1 |
| | | otherwise | 0 |

## FIG. 2

| Select patient sub-population | —100 |

| Enter clinical information about client | —102 |

| Acquire and analyze ECG wave segment | —104 |

| Compute probability of acute ischemia | —106 |

## FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4957115 A
- US 4998535 A
- US 5724983 A

### Non-patent literature cited in the description

- **Michael W. Pozen et al.** A Predictive Instrument to Improve Coronary-Care- Unit Admission Practices in Acute Ischemic Heart Disease. *The New England Journal of Medicine,* 17 May 1984, vol. 310, 1273-1278
- **H. P. Selker et al.** A Time-Insensitive Predictive Instrument for Acute Myocardial Infarction Mortality. *Med. Care,* 1991, vol. 29, 1196-1211
- **N. C. Cary.** *SUGI Supplement Library User's Guide,* 1983, 181-202
- **L. Engelman.** PLR Stepwise Logistic Regression. *BMDP Statistical Software,* 330-334
- **Will Penny et al.** Neural Networks in Clinical Medicine. *Medical Decision Making,* vol. 16 (4), 386-398
- **G.O. Stone.** An Analysis of the Delta Rule and the Learning of Statistical Associations. *Parallel Distributed Processing: Explorations in the Microstructure of Cognitions,* 1988, vol. 1, 444-459
- **D.E. Rumelhart et al.** Learning Internal Representations by Error Propagation. *Parallel Distributed Processing: Explorations in the Microstructure of Cognition,* 1988, vol. 1, 318
- Recursive Computation of Least Squares Estimators. *Regression and the Moore-Penrose Pseudoinverse,* 1972